# EUROPEAN PATENT SPECIFICATION

(11) **EP 1 262 181 B1**
(45) Date of publication and mention of the grant of the patent: **16.05.2007**
(21) Application number: 01908245.2
(22) Date of filing: 02.03.2001
(51) Int. Cl.: A61K 31/428, A61K 31/437, A61P 1/04, A61P 31/04

(54) **USE OF ISOTHIAZOLE DERIVATIVES AS UREASE INHIBITORS FOR THE TREATMENT OF HELICOBACTER PYLORI INDUCED GASTROINTESTINAL DISEASES**
VERWENDUNG VON ISOTHIAZOLDERIVATEN ALS UREASEINHIBITOREN ZUR BEHANDLUNG VON HELICOBACTER PYLORI VERURSACHTE GASTROINTESTINALE KRANKHEITEN
UTILISATION DE DERIVES DE L'ISOTHIAZOLE EN TANT QU' INHIBITEURS D'UREASE POUR LE TRAITEMENT DE MALADIES GASTRO-INTESTINALES GASTROINTESTINALES INDUITES PAR HELICOBACTER PILORI

(30) Priority: 07.03.2000 JP 2000062012
(43) Date of publication of application: 04.12.2002
(73) Proprietor: OTSUKA PHARMACEUTICAL CO., LTD., Chiyoda-ku Tokyo 101-8535 (JP)
(72) Inventor: KAJIWARA, Masahiro, Niiza-shi, Saitama 352-0016 (JP)
(74) Representative: HOFFMANN EITLE
(86) International application number: PCT/JP2001/001618
(87) International publication number: WO 2001/066112

(56) References cited:
- EP-A- 0 834 253
- JP-A- 7 101 862
- JP-A- 8 314 097
- JP-A- 10 316 651
- JP-A- 62 273 903
- PATENT ABSTRACTS OF JAPAN vol. 016, no. 289 (C-0956), 26 June 1992 (1992-06-26) & JP 04 077476 A (SANKYO CO LTD), 11 March 1992 (1992-03-11) & DATABASE WPI Section Ch, Week 199217 Derwent Publications Ltd., London, GB; Class B02, AN 1992-136747 & JP 04 077476 A (SANKYO CO LTD) 11 March 1992 (1992-03-11) & JP 04 077476 A (SANKYO CO LTD) 11 March 1992 (1992-03-11)
- DATABASE CHEMABS [Online] CHEMICAL ABSTRACTS SERVICE, COLUMBUS, OHIO, US; 12 May 1984 (1984-05-12), IMPICCIATORE, M. ET AL: "Biological properties of 1,2-benzisothiazoles. Pharmacological characteristics of 1,2-benzisoxazolin-3-one and 1,2-benzisothiazolin- 3-one" XP002292058 retrieved from STN Database accession no. 1972:254 & BOLLETTINO - SOCIETA ITALIANA DI BIOLOGIA SPERIMENTALE CODEN: BSIBAC; ISSN: 0037-8771, vol. 47, no. 10, 1971, pages 296-299,
- DATABASE MEDLINE [Online] US NATIONAL LIBRARY OF MEDICINE (NLM), BETHESDA, MD, US; February 1985 (1985-02), ANDERSEN K E ET AL: "Biocide patch tests." XP002292059 Database accession no. NLM3157538 & CONTACT DERMATITIS. FEB 1985, vol. 12, no. 2, February 1985 (1985-02), pages 99-103, ISSN: 0105-1873
- CHIYODA T. ET AL.: 'Convenient synthesis of 1,2-benzisothiazol-3(2H)-ones by cyclization reaction of acyl azide' SYNLETT. no. 10, October 2000, pages 1427 - 1428, XP002939735

## Description

### TECHNICAL FIELD

The present invention relates to a novel urease inhibitor and a novel anti-Helicobacter pylori agent.

### BACKGROUND ART

It has recently been made clear that urease produced by Helicobacter pylori has a close relation to the development of gastrointestinal diseases such as chronic gastritis and gastroduodenal ulcer. The mechanism of gastric mucosa injury due to urease is considered as follows.

Urea secreted from the gastric parietal is hydrolyzed by urease to produce ammonia and carbon dioxide. Ammonia has a strong mucosa injurious effect, thereby to cause a blood flow disorder of the gastric mucosa, and also neutralizes gastric acid, thereby to enable habitation of Helicobacter pylori within the stomach under a severe acidic environment. In case Helicobacter pylori adheres to the gastric mucosa, epithelial cells of the gastric mucosa produce Interleukin-8 (IL-8) as a kind of cytokines, while IL-8 acts on neutrophils, thereby to cause migration and activation of neutrophils. The activated neutrophils form phagocytosis and phagosome and also cause production of active oxygen and degranulation. The produced active oxygen itself causes a mucosa injury and induced to hypochlorous acid through an action of chlorine and myeloperoxidase in the stomach, and is also converted into monochloramine by means of ammonia, thus causing a cell injury.

It is also deemed that ammonia decreases reduced glutathione as a scavenger of active oxygen, thereby to enhance the production of active oxygen.

A substance having an action of inhibiting an activity of urease produced by Helicobacter pylori, namely, a urease activity inhibitor is effective to prevent and treat the development of gastrointestinal diseases such as gastric mucosa injury, and such a urease activity inhibitor has attracted special interest recently. Examples thereof include hydroxamic acids such as acetohydroxamic acid (A), benzohydroxamic acid (B), and nicotinohydroxamic acid (C); disulfides such as 2,2'-dipyridyl disulfide, cysteine, and disulfiram; and phenols such as hydroquinone, p-nitrophenol, and p-aminophenol.

Aforementioned hydroxamic acids (A) to (C) are reported in K. Kobayashi et al., Biochem. Biophys. Acta., 65, 380-383 (1962)) and K. Kobayashi et al., Biochem. Biophys. Acta., 227, 429-441 (1971)).

Disulfides are reported in R. Norris et al., Biochem. J., 159, 245-257 (1976)) and Matthew J. Todd, Robert P. Hausinger, J. Biol. Chem., 266, 10260-10267 (1991)).

However, these compounds are still insufficient in a urease activity inhibitory action of Helicobacter pylori and it is desired to study and develop a novel substance having a urease inhibitory action by which aforementioned compounds can be replaced in this field.

An object of the present invention is to provide a substance having a urease activity inhibitory action which is required in this field.

### DISCLOSURE OF THE INVENTION

The present inventors have intensively studied, heretofore, for the purpose of providing a novel urease inhibitory active substance which meets the requirements of this field and has previously succeeded in development of a series of novel dithiobenzohydroxamic acid derivatives which achieve the object. Thus, they have completed the invention based on this knowledge and filed the patent application (see Japanese Published Unexamined Patent Application (Kokai) Tokkyo Koho Hei No. 316651/1998).

In the subsequent study, the present inventors have found that several kinds of novel isothiazole derivatives have an excellent urease inhibitory active action and an excellent anti-Helicobacter pylori activity. Thus, they have completed the present invention based on this knowledge.

According to the present invention, there is provided a urease inhibitor which contains, as an active ingredient, an isothiazole derivative represented by the general formula (1) : 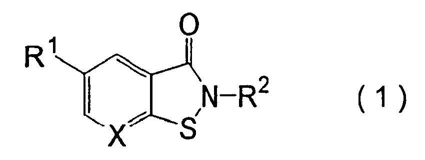 wherein R¹ represents a hydrogen atom or an amino group, R² represents a hydrogen atom, a lower alkyl group, or an acetyl group, and X represents a carbon atom or a nitrogen atom.

Also, according to the present invention, there is provided an anti-Helicobacter pylori agent which contains, as an active ingredient, an isothiazole derivative represented by the same general formula (1).

Furthermore, according to the present invention, there are provided the urease inhibitor and the anti-Helicobacter pylori agent, wherein the active ingredient is at least one kind selected from the group consisting of 1,2-benzoisothiazol-3(2H)-one, isothiazolo[5,4-b]pyridin-3(2H)-one, 5-amino-1,2-benzoisothiazol-3(2H)-one, N-methyl-1,2-benzoisothiazol-3(2H)-one and N-acetyl-1,2-benzoisothiazol-3(2H)-one.

### BRIEF DESCRIPTION OF THE DRAWINGS

Fig. 1 is a graph showing a urease inhibitory activity of the active ingredient compound of the present invention determined in accordance with Test Example 1.
Fig. 2 is a graph showing a urease inhibitory activity of the active ingredient compound of the present invention determined in accordance with Test Example 1.
Fig. 3 is a graph showing an anti-Helicobacter pylori activity of the active ingredient compound of the present invention determined in accordance with Test Example 2.

### BEST MODE FOR CARRYING OUT THE INVENTION

Among the compounds represented by the general formula (1) used as the active ingredient in the urease inhibitor and the anti-Helicobacter pylori agent of the present invention (hereinafter referredmerely to as "drug of the present invention", sometimes), a compound wherein R¹ and R² represent a hydrogen atom and X represents a carbon atom, namely, 1,2-benzoisothiazol-3(2H)-one (BIT) is a compound whose antimicrobial activity has conventionally been known. An antipsychotic activity has recently been found in a derivative derived from a carbonyl group at the 3-position thereof (F. Zini et al., Arch. Pharm., (Weinheim), 331, 219-223(1998); N. J. Hrib et al., J. Med. Chem., 15, 2308-2314 (1994); P. J. Collier et al., J. Appl. Bacteriol., 69, 567-577 (1990); J. P. Yevich et al., J. Med. Chem., 29, 359-369 (1986); R. Fisher et al., Arzeim Forsch., 14, 1301-1306 (1964)). As the method of preparing the derivative, for example, there is known a method of McClell and et al., comprising synthesizing the derivative from 2, 2-dithiodibenzoic acid via an acid chloride (E.W.McClelland et al., J. Chem. Soc., 3311-3315 (1926); L. Katz et al., J. Org. Chem., 19, 103-114 (1954)).

However, there has never been reported the fact that the compound has a urease activity, and the fact is the knowledge which is newly found out by the present inventors.

The compound represented by the general formula (1) as the active ingredient in the present invention, including BIT, and the method of preparing the same will be described in detail below.

In the present specification, examples of the lower alkyl group represented by R² in the general formula (1) include an alkyl group having 1 to 6 carbon atoms, such as methyl, ethyl, propyl, isopropyl, butyl, tert-butyl, pentyl, or hexyl group.

Examples of the compound represented by the general formula (1), which is preferred as the active ingredient of the drug of the present invention, include (1) those wherein R¹ is a hydrogen atom and (2) those wherein X is a carbon atom. Examples of other preferred compound include those wherein R¹ is an amino group, R² is a hydrogen atom and X is a carbon atom and those wherein both R¹ and R² are hydrogen atoms and X is a nitrogen atom.

Examples of the compound, which is particularly suited for medical use, include the following compounds: 1,2-benzoisothiazol-3(2H)-one, isothiazolo[5,4-b]pyridin-3(2H)-one, 5-amino-1,2-benzoisothiazol-3(2H)-one, N-methyl-1,2-benzoisothiazol-3(2H)-one, and N-acetyl-1,2-benzoisothiazol-3(2H)-one.

The compound used as the active ingredient can be prepared by aforementioned publicly known method or its equivalent. The present inventors have found an improved method capable of preparing the objective compound in higher yield with less side reaction product as compared with aforementioned publicly known method.

This improved method will be described in detail below, with respect to the kind of the substituent R² in the compound represented by the general formula (1).

The compound of the general formula (1) wherein R² is a hydrogen atom can be prepared by the following method via an acid azide.

Although the acid azide has conventionally been used to synthesize an amine, an amide and a thioester, these synthesis reactions are accompanied with the Curtius rearrangement, or employ the azide as an elimination group (E. Jabri et al., J. Mol. Biol., 227, 934-937 (1992)). However, the present inventors have achieved a method of obtaining a desired compound cyclized with the elimination of a nitrogen atom by generating azide thiosalicylate under low temperature conditions and conducting attack of a sulfur atom against azide nitrogen without causing the Curtius rearrangement.

According to the method, first of all, a compound of the general formula (1) wherein R¹ = R² = H and X = C can be prepared in the following manner. That is, publicly known thiosalicyclic acid as a starting material is reacted with diphenylphosphoryl azide (DPPA) in pyridine in the presence of triethylamine to obtain an acid azide as an intermediate, and then the acid azide is subjected to the cyclization reaction at room temperature.

In the reaction, conventionally used various bases can be used in place of triethylamine. Examples thereof include inorganic bases, for example, trialkylamine such as triethylamine or tributylamine; organic base such as pyridine, picoline, 1,5-diazabicyclo[4.3.0]nonene-5, 1,4-diazabicyclo[2.2.2]octane, or 1,8-diazabicyclo[5.4.0]undecene-7; alkali metal hydroxide such as sodium hydroxide or potassium hydroxide; alkali metal carbonate such as sodium carbonate or potassium carbonate; and alkali metal hydrogen carbonate such as sodium hydrogen carbonate or potassium hydrogen carbonate. It is suitable that these bases are used in an amount within a range from about 1 to 100 mol, and preferably from about 1 to 20 mol, per mol of the starting material compound.

Also other proper solvents can be used in place of pyridine. Examples thereof include hydrocarbon solvent such as benzene or hexane; ether solvent such as diethyl ether or tetrahydrofuran; and halogen solvent such as chloroform or methylene chloride.

DPPA used in aforementioned method can be replaced by the other azide such as NaN₃ or H₄N₂. When using NaN₃, carboxylic acid as the rawmaterial is preferably replaced by an acid halide thereof, commonly an acid chloride. When using H₄N₂, carboxylic acid as the raw material is replaced by esters thereof and, after the reaction, the desired acid azide can be derived by reacting with nitrous acid.

In the reaction, the amount of the azide such as DPPA or the like is usually selected within a range from about 1 to 10 mol, and preferably from about 1 to 2 mol, per mol of the starting material compound.

The reaction temperature of the reaction is generally selected within a range from about -10 to 10°C, and preferably from about -5 to 5°C, and the reaction is usually completed within a range from about 1 to 3 hours.

The subsequent cyclization reaction of the acid azide can be carried out by only returning the temperature of the reaction system to room temperature without isolating and purifying the acid azide from the reaction system.

Second, when using 2-mercaptonicotinic acid as the starting material, a compound of the general formula (1) wherein R¹ = R² = H and X = N can be prepared in the same manner as described above.

Third, when using 4-mercaptoisophthalic acid (and corresponding pyridine derivative) as the starting material, the desired compound of the general formula (1) wherein R¹ = NH₂ and R² = H can be prepared. That is, only one of two carboxyl groups of the starting material compound is selectively cyclized.

After the completion of the cyclization reaction, the residual acid azide of the resulting compound is subjected to the Curtius rearrangement reaction by heating under conditions acidified with hydrochloric acid and then subjected to the hydrolysis reaction, thus making it possible to obtain the desired 5-amino compound. More particularly, this Curtius rearrangement reaction is carried out by heating the resulting acid azide in a proper solvent, for example, hydrocarbon solvent such as benzene or hexane, ether solvent such as diethyl ether or tetrahydrofuran, halogen solvent such as chloroform or methylene chloride, and nitrogen-containing solvent such as pyridine at a temperature within a range from about 40 to 200°C for about 1 to 24 hours.

The subsequent hydrolysis reaction can be conducted in an aqueous solution of an acid such as hydrochloric acid or sulfuric acid or an aqueous solution of an alkali such as sodium hydroxide, potassium hydroxide or lithium hydroxide at a temperature within a range from about 0 to 100°C for 1 to 24 hours in accordance with a normal method.

In the third method, 4-mercaptoisophthalic acid, which is used as one of starting materials, can be prepared from a corresponding 4-bromo compound in the following manner.

That is, the 4-bromo compound is converted into an alkyl ester using alcohols such as ethyl alcohol capable of forming a proper alkyl ester, and then the alkyl ester is reacted with sodium sulfide, thereby replacing a bromo group by a mercapto group.

This reaction is completed by heating in a proper solvent (for example, hydrocarbon solvent such as alcohol solvent, benzene, or hexane; ether solvent such as diethyl ether or tetrahydrofuran; halogen solvent such as chloroform or methylene chloride; or nitrogen-containing solvent such as pyridine) at a temperature within a range from about 40 to 200°C, and preferably from about 80 to 150°C for about 1 to 24 hours using a NaSH, KSH, LiSH or SH gas in the amount within a range from about 1 to 100 mol, and preferably from about 1 to 20 mol, per mol of the raw compound.

The desired 4-mercaptoisophthalic acid can be prepared by hydrolyzing the 4-mercapto alkyl ester compound thus obtained. This hydrolysis reaction canbe conducted under the same conditions as those in case of aforementioned hydrolysis reaction.

Among the compounds represented by the general formula (1) as the active ingredient of the present invention, a compound wherein R² excludes a hydrogen atom can be prepared in the following manner using, as a raw material, a compound wherein R² is a hydrogen atom as an active ingredient of the present invention obtained by aforementioned method via the acid azide.

For example, a compound wherein R² is an alkyl group can be prepared by reacting a raw compound with a sulfuric acid dialkyl. Examples of the sulfuric acid dialkyl include corresponding sulfuric acid dialkyl such as sulfuric acid dimethyl or sulfuric acid diethyl. These sulfuric acid dialkyls are used in the amount within a range from about 1 to 20 mol, and preferably from about 1 to 10 mol, per mol of the raw compound. The reaction is conducted in a proper solvent, for example, aqueous alkali solution such as aqueous 10% sodium hydroxide solution, aqueous potassium hydroxide solution or aqueous lithium hydroxide solution, or in the absence of a solvent at a temperature within a range from about 0 to 100°C, and preferably from about 0 to 40°C, for about 1 to 24 hours.

Also a compound wherein R² is an acetyl group can be prepared by reacting a raw compound with an acetic anhydride. This reaction is usually conducted in a proper solvent such as pyridine, or in the absence of a solvent at a temperature within a range from about 0 to 100°C, and preferably from about 0 to 40°C, for about 1 to 24 hours using an acetic anhydride in the amount within a range from about 1 to 20, and preferably from 1 to 10 mol, per mol of a raw compound.

The objective compound obtained by the respective reactions described above can be isolated and purified from the reaction system by a conventional means. Examples of the means for isolation and purification include adsorption chromatography, recrystallization and solvent extraction.

The urease inhibitor and the anti-Helicobacter pylori agent of the present invention are prepared in the form of a general pharmaceutical preparation using the isothiazole derivative obtained as described above or its acid adduct salt, as an active ingredient, and a proper conventional carrier for preparation.

The acid adduct salt can be prepared by using a proper acid used commonly in formation of a salt, for example, inorganic acid such as hydrochloric acid, sulfuric acid, phosphoric acid, or hydrobromic acid, and organic acid such as oxalic acid, maleic acid, fumaric acid, malic acid, tartaric acid, citric acid, or benzoic acid in accordance with a normal method.

The carriers for preparation include commonly used diluents or excipients, for example, fillers, extenders, binders, humectants, disintegrators, surfactants and lubricants.

The form of the pharmaceutical preparation can be selected from various forms according to the therapeutic purpose, and typical examples thereof include tablets, pills, powders, liquid preparations, suspensions, emulsions, granules, and capsules.

When forming into the form of tablets, there can be used, as the carrier, excipients such as lactose, sucrose, sodium chloride, glucose, urea, starch, calcium carbonate, kaolin, crystalline cellulose, and silicic acid; binders such as water, ethanol, propanol, simple syrup, glucose solution, starch solution, gelatin solution, carboxymethylcellulose, shellac, methylcellulose, calcium phosphate, and polyvinyl pyrrolidone; disintegrators such as dry starch, sodium alginate, agar powder, laminaran powder, sodium hydrogen carbonate, calcium carbonate, polyoxyethylene sorbitan fatty acid esters, sodium lauryl sulfate, monoglyceride stearate, starch, and lactose; disintegration inhibitors such as sucrose, stearin, cacaobutter, and hydrogenated oil; absorption accelerators such as quaternary ammonium base and sodium lauryl sulfate; humectants such as glycerin and starch; adsorbents such as starch, lactose, kaolin, bentonite, and colloidal silicic acid; and lubricants such as purified talc, stearate, powdered boric acid, and polyethylene glycol.

Furthermore, tablets can optionally take the form of normal coated tablets, for example, sugar-coated tablet, gelatin-coated tablet, enteric coated tablet, film-coated tablet, two-layer tablet, and multi-layer tablet.

When forming into the form of pills, for example, excipients such as glucose, lactose, starch, cacao butter, hardened vegetable oil, kaolin, and talc; binders such as powdered arabic gum, powdered tragacanth, gelatin, and ethanol; and disintegrators such as laminaran and agar can be used as the carrier.

Capsules can be prepared by mixing various carriers described above with the derivative of the general formula (1) or its pharmaceutically acceptable salt and filling a hard gelatin capsule or soft gelatin capsule with the mixture.

If necessary, colorants, preservatives, perfumes, flavors, sweeteners and other pharmaceuticals can also be incorporated into the drug of the present invention.

The amount of the active ingredient to be incorporated into the pharmaceutical preparation of the present invention is not specifically limited and is appropriately selected from a wide range. The amount may be usually within a range from about 1 to 70% by weight based on the total amount of the pharmaceutical preparation.

The dose of the pharmaceutical preparation of the present invention is not specifically limited and is appropriately selected according to the age, sex and other conditions of the patient, state of the disease, and the form of various preparations. It is preferred that the pharmaceutical preparation is orally administered.

The dose of the pharmaceutical preparation of the present invention in human is appropriately selected according to the age, body weight, symptom, therapeutic effect, route of administration and treatment time, but the pharmaceutical preparation is preferably administered once or dividedly in two to several times in a day with a dairy dose within a range from about 0.1 to 100 mg/kg per one adult.

The pharmaceutical preparation of the present invention may be administered alone or in combination with other compounds as a pharmacologically active ingredient and pharmaceuticals containing the same, for example, antibiotics such as amoxycillin and clarithromycin; nitronidazole antiprotozoal agents such as metronidazole and tinidazole; antiulcer drugs such as bismuth preparation, sofalcone and plaunotol; and proton pump inhibitors such asomeprazole and lansoprazole. According to such combined administration, it is sometimes made possible to eradicate Helicobacter pylori with high probability and to more easily achieve complete recovery from gastrointestinal diseases caused such as chronic gastritis and gastroduodenal ulcer.

### EXAMPLES

To further illustrate the present invention in detail, Preparation Examples of the compounds of the present invention are described as Reference Examples and Test Examples of the compounds are then described.

### Reference Example 1

Preparation of 1,2-benzoisothiazol-3(2H)-one [compound of the general formula (1) wherein R¹ = R² = H and X = C, hereinafter referred to as "compound (3)"]

A solution of thiosalicyclic acid (3.1g, 20 mmol) in pyridine (40 ml) was added dropwise in a solution of diphenylphosphoryl azide (DPPA) (4.5ml, 20 mmol) in triethylamine (20 ml) at 0°C over 30 minutes.

The mixture was stirred at room temperature for 6 hours, followed by extraction with chloroform. The extract was washed with water and dried over anhydrous magnesium sulfate. After distillation, the residue was purified by silica gel column chromatography (ethyl acetate:hexane = 1:3) and recrystallized from chloroform to obtain 2.45 g of the objective compound (yield: 81%).
Melting point: 142-144°C
¹H-NMR (300MHz, DMSO-d₆)δ : 7.44(1H, dt, J=7.8, 0.9Hz), 7.64(1H, dt, J=7.8, 0.9Hz), 7.89(1H, dd, J=7.8, 0.9Hz), 7.99(1H, dd, J=7.8, 0.9Hz), 11.51 (1H, b)
¹³C-NMR(75MHz, DMSO-d₆)δ: 121.8, 124.4, 125.0, 125.1, 130.4, 147.7, 165.1
FT-IR(KBr) cm⁻¹: 606, 743, 1316, 1443, 1639, 2688, 2920, 3058
FAB-MS (m/z) : 152 (M+H) ⁺
Elemental analysis (%)
Calcd. for C₇H₅NOS: C, 55.61; H, 3.33; N, 9.26
Found: C, 55.37; H, 3.37; N, 9.25

### Reference Example 2

Preparation of 5-amino-1,2-benzoisothiazol-3(2H)-one [compound of the general formula (1) wherein R¹ = NH₂, R² = H and X = C, hereinafter referred to as "compound (10)"]

### (1) Preparation of 4-mercaptoisophthalic acid

4-bromoisophthalic acid (10.0 g, 40.8 mmol) was dissolved in concentrated sulfuric acid (10 ml) and dry ethyl alcohol (100 ml). The mixture was refluxed with boiling for one day, cooled to room temperature and neutralized with saturated sodium hydrogen carbonate, followed by extraction with chloroform. The extract was washed with water and dried over anhydrous magnesium sulfate. After distillation, the residue was dissolved in ethyl alcohol (100ml) and 70% sodium hydrogen sulfide (10 g, 0.13mol) was added.

The reaction mixture was refluxed with boiling for 2 hours and acidified with 50% hydrochloric acid, followed by extraction with diethyl ether. The extract was washed with water and dried over anhydrous magnesium sulfate, followed by distillation. The residue was purified by silica gel column chromatography (ethyl acetate:hexane = 1:20) and recrystallized from hexane to obtain 6.1 g of a 4-mercaptoisophthalic acid diethyl ester (58.8%).

The same compound (10 g, 39.3 mmol) was added to an aqueous 40% sodium hydroxide solution (100 ml) and the mixture was refluxed with boiling for 12 hours. After cooling, 50% hydrochloric acid was added at 0°C, thereby to produce a precipitate. The resulting precipitate was separated by filtration and washed with water. The precipitate was collected and recrystallized from methyl alcohol to obtain 7.61 g of the titled compound (yield: 97.7%). Melting point: 280°C or higher
¹H-NMR(300MHz, DMSO-d₆)δ: 3.17(1H, s), 7.64(1H, d, J=8.4Hz), 7.86(1H, dd, J=8.4, 1.8Hz), 8.47(1H, d, J=1.8Hz)
¹³C-NMR(75MHz, DMSO-d₆)δ: 126.8, 127.0, 131.8, 132.6, 146.1, 166.9, 167.5
FT-IR(KBr)cm⁻¹: 671, 761, 1256, 1305, 1412, 1598, 1683, 2545, 2641, 2830
FAB-MS(m/z): 199(M+H)⁺
Elemental analysis (%)
Calcd. for C₈H₆O₄S: C, 48.48; H, 3.05
Found: C, 48.03; H, 3.08

### (2) Preparation of 5-amino-1,2-benzoisothiazol-3(2H)-one (compound(10))

A solution of the compound (2.0 g, 10 mml) obtained in the item (1) in pyridine (20 ml) was added dropwise in a solution of diphenylphosphoryl azide (DPPA) (4.5 ml, 20 mmol) in triethylamine (10 ml) at 0°C over 30 minutes.

The mixture was stirred at room temperature for 12 hours and the reaction mixture was gradually added in 30% hydrochloric acid (50 ml). The mixture was refluxed with boiling for 6 hours and neutralized with saturated sodium hydrogen carbonate, and then the crude product was distilled and dissolved in methyl alcohol. The resulting solution was adsorbed on an acidic active alumina for chromatography. First, impurities were eluted and removed with methyl alcohol and then eluted with a mixed 20% concentrated ammonia water-methyl alcohol (1:4) solution to obtain a pure objective compound, which was crystallized from methyl alcohol (1.24 g, yield: 75%).
Melting point: 230-233°C
¹H-NMR(300MHz, DMSO-d₆) δ: 5.36 (1H, s), 6.96(1H, dd, J=8.5, 1.9Hz), 6.98(1H, d, J=1.9Hz), 7.55(1H, d, J=8.5Hz), 10.94(1H, br)
¹³C-NMR(75MHz, DMSO-d₆)δ: 164.5, 146.4, 125.36, 121.1, 119.4, 105.9
FT-IR(KBr)cm⁻¹: 611, 759, 1270, 1315, 1477, 1618, 2678, 2924, 2924, 3330, 3432
FAB-MS (m/z) : 16753(M+H)⁺
Elemental analysis (%)
Calcd. for C-7HrN₂OS: C, 50.59; H, 3.64; N, 16.86
Found: C, 50.80; H, 3.73; N, 16.79

### Reference Example 3

Preparation of isothiazolo[5,4-b]pyridin-3(2H)-one [compound of the general formula (1) wherein R¹ = R² = H and X = N, hereinafter referred to as "compound (11)"

A solution of 2-mercaptonicotinic acid (3.1 g, 20 mmol) in pyridine (40 ml) was added dropwise in a solution of diphenylphosphoryl azide (DPPA) (4.5ml, 20mmol) in triethylamine (20 ml) at 0°C over 30 minutes.

The mixture was stirred at room temperature for 4 hours and the reaction mixture was distilled and adsorbed on an acidic active alumina for chromatography. First, impurities were eluted and removed with methyl alcohol and then eluted with 10% acetic acid-methyl alcohol to obtain a pure objective compound, which was crystallized from methyl alcohol (2.64 g, yield: 87%).
Melting point: 235-237°C
¹H-NMR(300MHz, DMSO-d₆)δ: 7.51(1H, d, J=4.8Hz), 7.53(1H, d, J=5.1Hz), 8.33(1H, dd, J=5.1, 1.5Hz), 8.83(1H, dd, J=4.8, 1.5Hz), 11.93(1H, b)
¹³C-NMR (75MHz, DMSO-d₆)δ: 118.9, 120.8, 133.5, 153.0, 163.
FT-IR(KBr)cm⁻¹: 606, 753, 1387, 1461, 1674, 2696, 2911, 3032
FAB-MS(m/z):153(M+H)⁺
Elemental analysis (%)
Calcd. for C₆H₄N₂OS : C, 47.36; H, 2.65; N, 18.41
Found: C, 47.26; H, 2.68; N, 18.11

### Reference Example 4

Preparation of N-methyl-1,2-benzoisothiazol-3(2H)-one [compound of the general formula (1) wherein R¹ = H, R² = methyl group and X = S, hereinafter referred to as "compound (12)"

An aqueous 10% sodium hydroxide solution (20 ml) of 1, 2-benzoisothiazol-3(2H) -one (0.5g, 3.3 mmol) was added dropwise in dimethylsulfuric acid (2 ml, 21 mmol). The mixture was refluxed with boiling overnight, followed by extraction with chloroform. The extract was washed with 10% hydrochloric acid, dried over anhydrous magnesium sulfate and then distilled. The residue was recrystallized from ethyl acetate to obtain 0.47 g of the objective compound (yield: 86%).
Melting point: 43-46°C
¹H-NMR(300MHz, CDCl₃) δ: 3.45(3H, s), 7.41(1H, dt, J=8.2, 1.4Hz), 7.52-7.64(2H, m), 8.04(1H, dt, J=8.0, 0.5Hz)
¹³C-NMR(75MHz, CDCl₃)δ: 30.4, 120.3, 124.5, 125.5, 126.7, 131.8, 140.0, 165.6
FT-IR(KBr)cm⁻¹: 670, 745, 1341, 1447, 1638, 3449
FAB-MS(m/z): 166(M+H)⁺
Elemental analysis (%)
Calcd. for C₈H₇NOS: C, 58.16; H, 4.27; N, 8.48
Found: C, 57.48; H, 4.26; N, 8.38

### Reference Example 5

Preparation of N-acetyl-1,2-benzoisothiazol-3(2H)-one [compound of the general formula (1) wherein R¹ = H, R² = acetyl group and X = S, hereinafter referred to as a "compound (13)"

A solution of 1,2-benzoisothiazol-3(2H)-one (1 g, 6.6 mmol) in pyridine (20 ml) was added dropwise in acetic anhydride (10 ml). The mixture was stirred at room temperature overnight, followed by extraction with chloroform. The extract was washed with saturated sodium hydrogen carbonate, dried over anhydrous magnesium sulfate and then distilled. The residue was purified by silica gel column chromatography (ethyl acetate:hexane = 1:10) and recrystallized from ethyl acetate to obtain 1.2 g of the objective compound (yield: 94%).
Melting point: 135-137°C
¹H-NMR(300MHz, CDCl₃)δ: 2.79(3H, s), 7.41(1H, ddd, J=8.0, 7.1, 0.8Hz), 7.53(1H, dt, J=8.0, 0.8Hz), 7.71(1H, ddd, J=8.0, 7.1, 1.4Hz), 8.03(1H, ddd, J=8.0, 1.4, 0.8Hz)
¹³C-NMR(75MHz, CDCl₃)δ: 24.9, 120.6, 125.4, 125.9, 127.9, 134.5, 141.0, 163.4, 170.1
FT-IR(KBr)cm⁻¹: 604, 737, 1368, 1451, 1687, 2931, 3009, 3064 EI-MS(m/z)(rel.int.%): 193(M⁺, 16), 151(100), 123(5), 96(4)
Elemental analysis (%)
Calcd. for C₉H₇NO₂S: C, 55.95; H, 3.65; N, 7.25
Found: C, 55.92; H, 3.66; N, 7.20

### Test Example 1

### Urease activity inhibition test

Using the compound as an active ingredient of the present invention as the test compound (urease inhibitor sample), the following urease activity inhibitory test was conducted.

Using ¹³C-urea as a substrate, the amount of urea, which is eliminated due to the urease enzyme reaction, was measured with a lapse of time by means of ¹³C-NMR. On the basis of an elimination rate (M/sec) of ¹³C-urea in the absence of the urease inhibitor sample, the case where the elimination rate in the presence of the sample is reduced to half was calculated as IC₅₀ of the sample.
[¹³C-NMR apparatus and measuring conditions]
Apparatus: GEMINI300 (75 MHz) manufactured by Varian Co.
Acquisition time: 1.0 seconds
Pulse decay time: 0.5 seconds
Scan number: 8-30 times
Probe temperature: 20°C
Spectral band width: 18102.9 Hz
Date point: 36192 pulse
Angle: 27°
(Preparation of sample) In a NMR tube having a diameter of 5 mm, Helicobacter pylori urease (manufactured by OTSUKA PHARMACEUTICAL CO., 1.6 units) dissolved in 500 µl of a mixed solvent (pH7) of 400 µl of 0.1M phosphate buffer (pH7) and 100 µl of DMSO was added and each test compound (urease inhibitor sample) dissolved in 100 *µ*l of ethanol was added, followed by standing at 20°C for 30 minutes and further standing on an ice bath for 10 minutes.

¹³C-urea (manufactured by Mass Trace, Inc. (99 atomic % ¹³C), 1 mg) dissolved separately in 100 µl of the same mixed solvent cooled to 0°C was added in the NMR tube, followed by rapid shaking to prepare a sample wherein the reaction solvent amount is 600 µl. A sample containing no test compound was used as a control.

### [Measuring procedure]

Each sample was inserted into a probe and the enzyme reaction was conducted at the reaction temperature (temperature of the probe) of 20°C and the measurement was conducted every 20 seconds after 1 to 4 minutes have passed since the beginning of the reaction, while the measurement was conducted every one minute after 4 or more minutes have passed, using a ¹³C-NMR apparatus. Consequently, an elimination rate (M/sec) of a signal (165 ppm) of ¹³C-urea as the substrate was determined.

### [Results]

Using the compound (BIT) prepared in Reference Example 1 as the test compound, a test was conducted. As a result, IC₅₀ of BIT was 5.5 × 10⁻⁵ M.

The same test was repeated, except for using a commercially available jack bean urease in place of the Helicobacter pylori urease. As a result, IC₅₀ of BIT was 13.2×10⁻⁵ M.

These values are equivalent to those of hydroxamic acids as a conventionally known typical urease inhibitor (K. Kyoichi et al., Biochim. Biophys. Acta, 65, 380-383 (1962); K. Kyoichi et al., ibid, 227, 429-441 (1971); S. Odake et al., Biol. Pharm. Bull., 17, 1329-1332 (1994)) and this fact shows that BIT has a strong urease inhibitory activity.

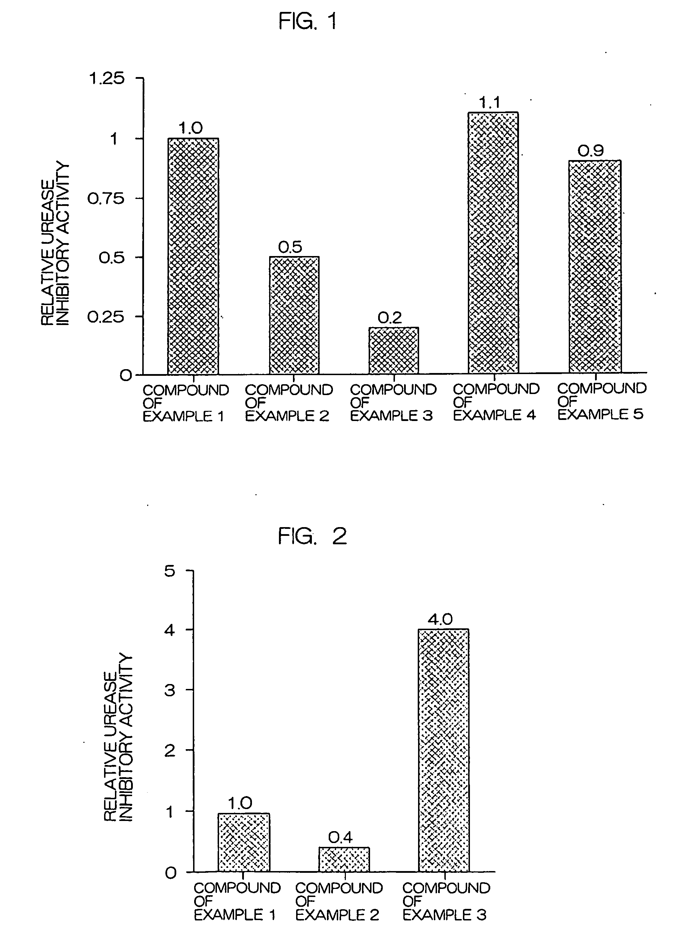

Using the respective compounds obtained in Reference Examples 2 to 5 as the test compound, the same test (using the jack bean urease) was repeated and IC₅₀ of each test compounds was determined, thus calculating a relative urease inhibitory activity value relative to a standard (1) for the same value of BIT. The results are shown in Fig. 1.

In Fig. 1, the ordinate shows a relative urease inhibitory activity of the respective test compounds (compounds obtained in Reference Examples 2 to 5) relative to the value 1 for the urease inhibitory activity of the compound (BIT) obtained in Reference Example 1, while the abscissa shows the respective test compounds.

Furthermore, the same test was repeated, except for adjusting the pH of the reaction system to 6 (a mixed solution of µ 1 of a solution prepared by dissolving NaH₂PO₄·2H₂O (1.04 g) in distilled water to make 50 ml, 150 µl of a solution prepared by dissolving Na₂HPO₄·12H₂O (2.39 g) in distilled water to make 50 ml, 100 µl 1 of a solution prepared by dissolving NaH₂PO₄·2H₂O (1.04 g) and H₃PO₄ (100 µl) in distilled water to make 50 ml and 100 µl of DMSO was used as buffer (pH6)). The results are shown in Fig. 2 (with respect to the compounds obtained in Reference Examples 2 and 3).

As is apparent from Fig. 1 and Fig. 2, any of the compounds as the active ingredient of the present invention prepared in Reference Examples 2 to 5 has an excellent urease inhibitory activity similar to the same compound as the active ingredient prepared in Reference Example 1.

### Test Example 2

### Anti-Helicobacter pylori activity test

An anti-Helicobacter pylori activity test of the compound as an active ingredient of the present invention was conducted in the following procedure using a dilution method.

### (1) Preparation of Helicobacter pylori solution

Helicobacter pylori ATCC 43504 strains were innoculated in a medium in petri dish (Brucella agar (BECTON DIKINSON), containing 7% FBS) and then cultured in a mixed carbonic acid gas-nitrogen gas culture medium (10% CO₂, 5% O₂, 85% N₂, 37°C) for 2 days. The strains were recovered, cultured similarly in a liquidmediumusing a Brucella broth (BECTON DIKINSON) for one day and then diluted with the same medium to control OD₆₆₀ₙₘ to 0.1.

### (2) Preparation of dilution series of test compound

The test compound was dissolved in 50% ethanol-saline to prepare a 1 mg/ml solution and the solution was 1- to 2048-fold diluted by 12 steps with the same medium to prepare dilution series.

### (3) Test procedure

In each well of cell culture plate (20 µl/well), each stepwisely diluted solution (20 l) of the test compound was charged and a Brucella medium (containing 7% FBS) (160 µl) was added, and then a pylori strain solution (20 µl) was finally added. After the plate was cultured in a mixed carbonic acid gas-nitrogen gas culture medium (10% CO₂, 5% O₂, 85% N₂) at 37°C for 3 days, the turbidity (OD₆₆₀ₙₘ) of each well was measured and the bacterial inhibitory rate (eradication rate, %) was calculated using the same value upon beginning of the test as a standard.

### (4) Results

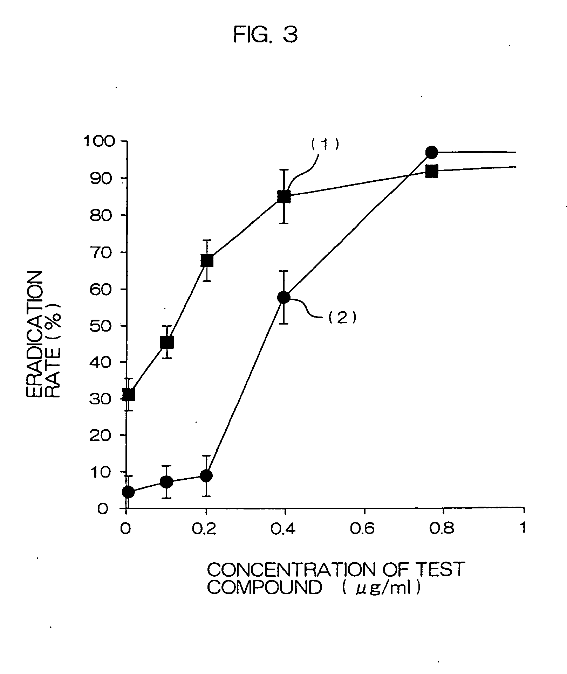

The results obtained by using the active ingredient compound of the present invention obtained in Reference Example 1 are shown in Fig. 3. In Fig. 3, the ordinate shows the eradication rate (%), while the abscissa shows the concentration (µg/ml) of the test compound. In Fig. 3, plots (1) show the results obtained by using the compound as an active ingredient of the present invention, while plots (2) show the results obtained by using the following control compound.

Control compound: Metronidazole (MN), which has conventionally been known as an agent for eradicating Helicobacter pylori, was used.

The above test results were expressed in mean ±SD. In case of the active ingredient compound of the present invention, n = 12. In case of the control compound, n = 6.

As is apparent from Fig. 3, the compounds as the active ingredient of the present invention (obtained in Reference Example 1) exhibits higher Helicobacter pylori inhibitory rate at a lower concentration as compared with MN and, therefore, it has an anti-Helicobacter pylori activity stronger than that of MN.

As described above, it is apparent that any of the compounds as the active ingredient of the present invention has both a urease inhibitory activity and an anti-Helicobacter pylori activity.

### INDUSTRIAL APPLICABILITY

Thus, according to the present invention, a compound having an excellent inhibitory activity against urease of Helicobacter pylori as well as a urease inhibitor and an anti-Helicobacter pylori agent, which contain the compound as an active ingredient, are provided. The drugs of the present invention are effective to prevent and treat gastrointestinal diseases caused by urease of Helicobacter pylori, such as chronic gastritis and gastroduodenal ulcer.

## Claims

1. Use of an isothiazole derivative represented by the general formula (1) : 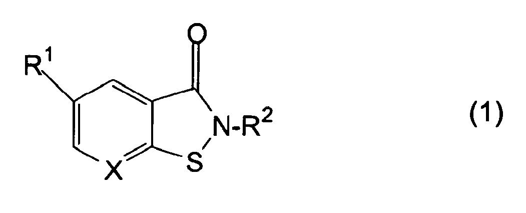 wherein R¹ represents a hydrogen atom or an amino group, R² represents a hydrogen atom, a lower alkyl group, or an acetyl group, and X represents a carbon atom or a nitrogen atom, as an active ingredient for the preparation of a medicament for the treatment and prevention of a gastrointestinal disease caused by urease which is produced by Helicobacter pylori.

2. Use according to claim 1, wherein the active ingredient is at least one kind selected from the group consisting of 1,2-benzoisothiazol-3(2H)-one, isothiazolo[5,4-b]pyridin-3(2H)-one, 5-amino-1,2-benzoisothiazol-3(2H)-one, N-methyl-1,2-benzoisothiazol-3 (2H)-one and N-acetyl-1,2-benzoisothiazol-3(2H)-one.

3. Use of an isothiazole derivative represented by the general formula (1): 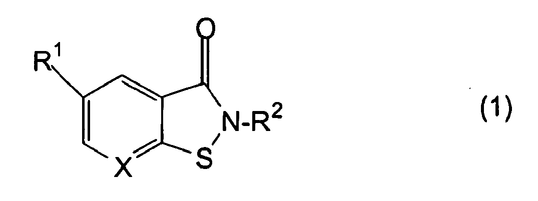 wherein R¹ represents a hydrogen atom or an amino group, R² represents a hydrogen atom, a lower alkyl group, or an acetyl group, and X represents a carbon atom or a nitrogen atom, as an active ingredient for the preparation of a medicament for the treatment and prevention of a gastrointestinal disease caused by Helicobacter pylori.

4. Use according to claim 3, wherein the active ingredient is at least one kind selected from the group consisting of 1,2-benzoisothiazol-3(2H)-one, isothiazolo[5, 4-b]pyridin-3 (2H) -one, 5-amino-1,2-benzoisothiazol-3(2H)-one, N-methyl-1,2-benzoisothiazol-3(2H)-one and N-acetyl-1,2-benzoisothiazol-3(2H)-one.

## Patentansprüche

1. Verwendung eines Isothiazolderivats dargestellt durch die allgemeine Formel(1): 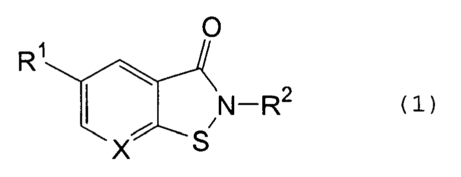 worin R¹ ein Wasserstoffatom oder eine Aminogruppe darstellt, R² ein Wasserstoffatom, eine Niederalkylgruppe oder eine Acetylgruppe darstellt, und X ein Kohlenstoffatom oder ein Stickstoffatom darstellt,
als Wirkstoff für die Herstellung eines Medikaments für die Behandlung und Verhinderung einer Gastrointestinalkrankheit, die durch Urease, die von Helicobacter pylori erzeugt wird, hervorgerufen wird.

2. Verwendung gemäß Anspruch 1, wobei der Wirkstoff zumindest eine Art ausgewählt aus der Gruppe bestehend aus
1,2-Benzoisothiazol-3(2H)-on,
Isothiazolo[5,4-b]pyridin-3(2H)-on,
5-Amino-1,2-benzoisothiazol-3(2H)-on,
N-Methyl-1,2-benzoisothiazol-3(2H)-on und N-Acetyl-1,2-benzoisothiazol-3(2H)-on ist.

3. Verwendung eines Isothiazolderivats dargestellt durch die allgemeine Formel (1): 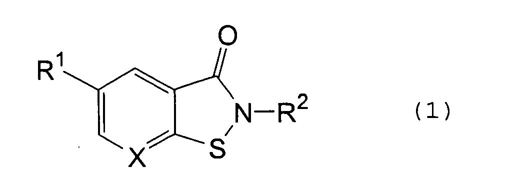 worin R¹ ein Wasserstoffatom oder eine Aminogruppe darstellt, R² ein Wasserstoffatom, eine Niederalkylgruppe oder eine Acetylgruppe darstellt, und X ein Kohlenstoffatom oder ein Stickstoffatom darstellt,
als Wirkstoff für die Herstellung eines Medikaments für die Behandlung und Verhinderung einer durch Helicobacter pylori hervorgerufenen Gastrointestinalkrankheit.

4. Verwendung gemäß Anspruch 3, wobei der Wirkstoff zumindest eine Art ausgewählt aus der Gruppe bestehend aus
1,2-Benzoisothiazol-3(2H)-on,
Isothiazolo[5,4-b]pyridin-3(2H)-on,
5-Amino-1,2-benzoisothiazol-3(2H)-on,
N-Methyl-1,2-benzoisothiazol-3(2H)-on und
N-Acetyl-1,2-benzoisothiazol-3(2H)-on ist.

## Revendications

1. Utilisation d'un dérivé d'isothiazole représenté par la formule générale (1) : 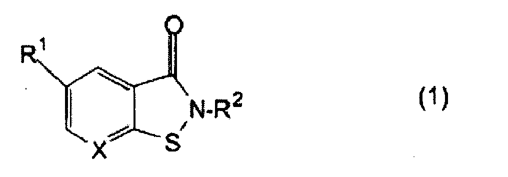 dans laquelle R¹ représente un atome d'hydrogène ou un groupe amino, R² représente un atome d'hydrogène, un groupe alkyle inférieur, ou un groupe acétyle, et X représente un atome de carbone ou un atome d'azote, en tant qu'ingrédient actif pour la préparation d'un médicament pour le traitement et la prévention d'une maladie gastro-intestinale provoquée par une uréase qui est produite par Helicobacter pylori.

2. Utilisation selon la revendication 1, dans laquelle l'ingrédient actif est au moins un type choisi dans le groupe constitué par
la 1,2-benzoisothiazol-3(2H)-one,
l'isothiazolo[5,4-b]pyridin-3(2H)-one,
la 5-amino-1,2-benzoisothiazol-3(2H)-one,
la N-méthyl-1,2-benzoisothiazol-3(2H)-one et
la N-acétyl-1,2-benzoisothiazol-3(2H)-one.

3. Utilisation d'un dérivé d'isothiazole représenté par la formule générale (1) : 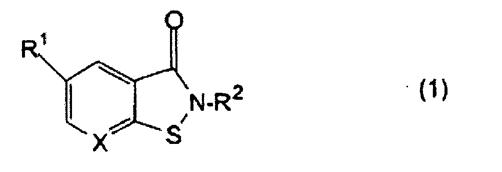 dans laquelle R¹ représente un atome d'hydrogène ou un groupe amino, R² représente un atome d'hydrogène, un groupe alkyle inférieur, ou un groupe acétyle, et X représente un atome de carbone ou un atome d'azote, en tant qu'ingrédient actif pour la préparation d'un médicament pour le traitement et la prévention d'une maladie gastro-intestinale provoquée par Helicobacter pylori.

4. Utilisation selon la revendication 3, dans laquelle l'ingrédient actif est au moins un type choisi dans le groupe constitué par
la 1,2-benzoisothiazol-3(2H)-one,
l'isothiazolo [5,4-b] pyridin-3(2H)-one,
la 5-amino-1,2-benzoisothiazol-3(2H)-one,
la N-méthyl-1,2-benzoisothiazol-3(2H)-one et
la N-acétyl-1,2-benzoisothiazol-3(2H)-one.
